(19) Europäisches Patentamt
European Patent Office
Office européen des brevets

(11) EP 1 924 242 B1

(12) **EUROPEAN PATENT SPECIFICATION**

(45) Date of publication and mention of the grant of the patent:
**03.11.2010 Bulletin 2010/44**

(21) Application number: **06779229.1**

(22) Date of filing: **29.08.2006**

(51) Int Cl.:
***A61K 9/00*** *(2006.01)* ***A61K 9/50*** *(2006.01)*
***A61K 9/16*** *(2006.01)*

(86) International application number:
**PCT/GB2006/003199**

(87) International publication number:
**WO 2007/026138 (08.03.2007 Gazette 2007/10)**

(54) **PHARMACEUTICAL COMPOSITION COMPRISING ANASTROZOLE**

PHARMAZEUTISCHE ZUSAMMENSETZUNG ENTHALTEND ANASTROZOL

COMPOSITION PHARMACEUTIQUE COMPRENANT ANASTROZOLE

(84) Designated Contracting States:
**AT BE BG CH CY CZ DE DK EE ES FI FR GB GR HU IE IS IT LI LT LU LV MC NL PL PT RO SE SI SK TR**

(30) Priority: **31.08.2005 GB 0517674**

(43) Date of publication of application:
**28.05.2008 Bulletin 2008/22**

(73) Proprietor: **AstraZeneca AB**
**151 85 Södertälje (SE)**
Designated Contracting States:
**AT BE BG CH CY CZ DE DK EE ES FI FR GB GR HU IE IS IT LI LT LU LV MC NL PL PT RO**

(72) Inventors:
- **GELLERT, Paul, Richard**
**Macclesfield Cheshire SK10 4TG (GB)**
- **MATHARU, Balvinder, Singh**
**Macclesfield Cheshire SK10 4TG (GB)**

(74) Representative: **Giles, Allen Frank et al**
**AstraZeneca AB,**
**Global Intellectual Property**
**151 85 Södertälje (SE)**

(56) References cited:
**EP-A- 1 291 023** **WO-A-96/38133**
**US-A1- 2003 129 233** **US-B1- 6 217 911**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The present invention relates to slow release anastrozole formulations, more particularly to biodegradable polymers, typically polylactide polymers and poly(lactide-co-glycolide) co-polymers, in which anastrozole is incorporated, including microparticle formulations and monolithic implant formulations. The invention also relates to said formulations for use in methods of treatment, particularly for use in methods for the treatment of breast cancer.

Anastrozole (Arimidex™) is an aromatase inhibitor, aromatase inhibitors are a class of compounds that act to inhibit oestrogen synthesis in tissues. These compounds prevent oestrogen biosynthesis by inhibiting the enzyme aromatase, which catalyses the conversion of adrenal androgens (androstenedione and testosterone) to oestrogens (oestrogen and oestradiol). Anastrozole is a non-steroidal aromatase inhibitor which is highly selective, well tolerated and is effective in treating advanced breast cancer (Buzdar ct al 1995, 5 The Breast 4(3) : 256-257 Abs 104; Jonat et al 1995, European Journal of Cancer 32A(3): 404-412; Plourde et al 1995, Journal of Steroid Biochemistry 53:175-179). (Further information on the clinical experience with Arimidex can be found in the prescribing information sheet for Arimidex). Anastrozole is described in U.S. patent RE 366717.

A number of prolonged release formulations using polylactide and poly(lactide-co-glycolide) co-polymers are known. For example, EP 058 481 describes monolithic implants comprising a biodegradable poly(lactide-co-glycolide) co-polymer and an LH-RH agonist.

Anastrozole is currently given as an immediate release 1mg oral tablet daily. It is important that patients take anastrozole daily to receive the optimum therapeutic benefit. Patient compliance with such a daily dosing regimen is however, difficult to ensure, especially where the course of therapy is long or of intermediate or lifetime duration. Thus, there is a need for a prolonged release formulation of anastrozole to improve patient compliance/convenience and give patients optimum therapeutic benefit. Formulating compounds in prolonged release dosage forms, such as monolithic implants and microparticle formulations also provides other benefits. For example, less frequent dosing of drugs in the form of prolonged release formulations effectively smoothes out fluctuations in the plasma concentration-time profile. Such smoothing out of plasma profiles has the potential to not only improve the therapeutic effect of the drug, but also to reduce any unwanted side effects. A further advantage of a prolonged release formulation, particularly important for oncology indications, is the improvement in 'quality of life' it gives by removing the daily reminder of the disease.

In order to gain patient acceptance of such an implant or microparticle formulation over the conventional oral treatment, it is important that the formulation causes minimal discomfort on injection. It would thus be highly advantageous to administer the dose in as small as possible amount of formulation, i.e. low injection volume of microparticles or small size of implant. In order to achieve this at the oral dose of 1mg/day, the formulation would need to contain a high weight percentage of anastrozole. However, anastrozole has a molecular weight of 293.4 Daltons and a water solubility of 0.53mg/ml at 25°C. Low molecular weight compounds with such solubility are not ideally suited to the formation of prolonged release formulations. This is because low levels of drug delivery *in vivo* with large initial drug bursts and only short periods of drug release are typically expected for microparticle or implant formulations comprising such compounds. The effect is potentiated when formulations contain high drug levels and low levels of rate modifying polymer. Furthermore, many of the most established manufacturing processes used to prepare prolonged release microparticle formulations employ aqueous solvents. For these processes, low molecular weight water-soluble compounds, such as anastrozole tend to partition into these aqueous phases during the encapsulation process leading to poor encapsulation efficiencies and low drug loadings.

We have surprisingly found that it is possible to produce prolonged release formulations of anastrozole using polylactide and poly(lactide-co-glycolide) co-polymers which release anastrozole over periods of at least 10 days, when placed in an aqueous physiological-type environment.

According to a first aspect of the present invention there is provided a prolonged release formulation comprising:

(i) a polylactide polymer or poly(lactide-co-glycolide) co-polymer having an average molecular weight of from 5,000 Daltons to 50,000 Daltons;
(ii) a molar ratio of lactide to glycolide of between 100:0 to 50:50; and
(iii) from about 10 to 55% by weight of anastrozole based upon the total weight of the formulation,

wherein the prolonged release formulation continuously releases anastrozole over a period of at least 10 days when placed in an aqueous physiological-type environment.

According to a further aspect of the present invention there is provided a prolonged release formulation comprising:

(i) a polylactide polymer or poly(lactide-co-glycolide) co-polymer having an average molecular weight of from 5,000 Daltons to 50,000 Daltons;
(ii) a ratio of lactide to glycolide of between 100:0 to 50:50; and

(iii) from about 10 to 55% by weight of anastrozole based upon the total weight of the formulation;

wherein
(i) for anastrozole loadings at lactide to glycolide ratios given in the table below the average molecular weight of the polymer is above the figure given in the table:

| Lactide/Glycolide | Loading | Average Molecular Weight |
|---|---|---|
| 50:50- 65:35 | >=10% | >15,000 |
| 50:50- 65:35 | >20% | >25,000 |
| 65:35 | >30% | >45,000 |
| 66:34 - 75:25 | >20% | >15,000 |
| 66:34 - 75:25 | >30% | >40,000 |
| 76:24 - 85:15 | >20% | >10,000 |
| 76:24 - 85:15 | >30% | >30,000 |
| 85:15 | >40% | >45,000 |
| 86:14 - 95:5 | >20% | >10,000 |
| 86:14 - 95:5 | >30% | >15,000 |
| 86:14 - 95:5 | >35% | >30,000 |
| 86:14 - 95:5 | >40% | >40,000 |
| 96:4 - 100/0 | >30% | >17,000 |
| 96:4 - 100/0 | >40% | >35,000 |

and
(ii) for anastrozole loadings above 30% the lactide/glycolide ratio is 65:35 or above and for anastrozole loadings above 40% the lactide/glycolide ratio is 85:15 or above.

According to a further aspect of the present invention there is provided a prolonged release formulation comprising:

(i) a polylactide polymer or poly(lactide-co-glycolide) co-polymer having an average molecular weight of from 5,000 Daltons to 50,000 Daltons;
(ii) a ratio of lactide to glycolide of between 100:0 to 50:50; and
(iii) from about 10 to 55% by weight of anastrozole based upon the total weight of the formulation;

wherein
(i) for anastrozole loadings at lactide to glycolide ratios given in the table below the average molecular weight is above the figure given in the table:

| Lactide/Glycolide | Loading | Average. Molecular Weight |
|---|---|---|
| 50:50 - 65:35 | >=10% | >15,000 |
| 50:50 - 65:35 | >20% | >25,000 |
| 66:34 - 75:25 | >20% | >15,000 |
| 76:24 - 85:15 | >20% | >10,000 |
| 86:14 - 95:5 | >30% | >15,000 |

and
(ii) for anastrozole loadings above 30% the lactide/glycolide ratio is 65:35 or above and for anastrozole loadings above 40% the lactide/glycolide ratio is 85:15 or above.

For example, for anastrozole loadings of above 20% with a lactide/glycolide ratio between 50:50 and 65:35 the

molecular weight of the polymer or co-polymer is above 25,000 Daltons.

According to a further aspect of the present invention there is provided a prolonged release formulation comprising:

(i) a polylactide polymer or poly(lactide-co-glycolide) co-polymer having an average molecular weight of from 5,000 Daltons to 50,000 Daltons;
(ii) a ratio of lactide to glycolide of between 100:0 to 50:50; and
(iii) from about 10 to 55% by weight of anastrozole based upon the total weight of the formulation;

wherein
the minimum average molecular weight of the polymer can be calculated from the equation below:

$$MW = -71.88 + 25.0(0.5 + \tanh(14.3(f_A - 0.33))) + 84.5(f_G + 1.08) - 12.8(f_G + 1.08)^2$$

wherein
$f_A$ is the fraction of anastrozole wherein 1.0 is 100%, i.e. 5% would be expressed as 0.05; and
$f_G$ is the fraction of glycolide wherein 1.0 is 100%, i.e. 50% would be expressed as 0.50.

According to a further aspect of the present invention there is provided a prolonged release formulation comprising:

(i) a polylactide polymer or poly(lactide-co-glycolide) co-polymer having an average molecular weight of from 5,000 Daltons to 50,000 Daltons;
(ii) a molar ratio of lactide to glycolide of between 100:0 to 50:50; and
(iii) from about 10 to 65% by weight of anastrozole based upon the total weight of the formulation;

wherein the prolonged release formulation is a monolithic implant and the prolonged release formulation continuously releases anastrozole over a period of at least 10 days when placed in an aqueous physiological-type environment.

According to a further aspect of the present invention there is provided a prolonged release formulation comprising:

(i) a polylactide polymer or poly(lactide-co-glycolide) co-polymer having an average molecular weight of from 5,000 Daltons to 50,000 Daltons;
(ii) a ratio of lactide to glycolide of between 100:0 to 50:50; and
(iii) from about 10 to 65% by weight of anastrozole based upon the total weight of the formulation;

wherein the prolonged release formulation is a monolithic implant and wherein for anastrozole loadings at lactide to glycolide ratios given in the table below the average molecular weight is above the figure given in the table:

| Lactide/Glycolide | Loading | Average Molecular Weight |
|---|---|---|
| 50:50- 65:35 | >15% | >10,000 |
| 50:50- 65:35 | >25% | >15,000 |
| 50:50- 65:35 | >40% | >20,000 |
| 66:34 - 75:25 | >20% | >10,000 |
| 66:34 - 75:25 | >40% | > 15,000 |
| 76:24 - 85:15 | >40% | >15,000 |

According to a further aspect of the present invention there is provided a prolonged release formulation comprising:

(iv) a polylactide polymer or poly(lactide-co-glycolide) co-polymer having an average molecular weight of from 5,000 Daltons to 50,000 Daltons;
(v) a ratio of lactide to glycolide of between 100:0 to 50:50; and

(vi) from about 10 to 65% by weight of anastrozole based upon the total weight of the formulation;

wherein the prolonged release formulation is a monolithic implant and wherein the minimum average molecular weight of the polymer can be calculated from the equation below:

$$MW = 9.937 - 13.0(f_A+0.547) + 5.684(f_A+0.547)^3 - 9.89(f_G-0.177) + 78.95(f_G-0.177)^3 + 83.25(f_A*f_G)$$

wherein
$f_A$ is the fraction of anastrozole wherein 1.0 is 100%, i.e. 5% would be expressed as 0.05; and
$f_G$ is the fraction of glycolide wherein 1.0 is 100%, i.e. 50% would be expressed as 0.50.

According to a further aspect of the present invention there is provided a prolonged release formulation comprising:

(i) a polylactide polymer or poly(lactide-co-glycolide) co-polymer having an average molecular weight of from 5,000 Daltons to 50,000 Daltons;
(ii) a molar ratio of lactide to glycolide of between 100:0 to 50:50; and
(iii) from about 10 to 55% by weight of anastrozole based upon the total weight of the formulation;

wherein the prolonged release formulation is a microparticle formulation and the prolonged release formulation continuously releases anastrozole over a period of at least 10 days when placed in an aqueous physiological-type environment.

According to a further aspect of the present invention there is provided a prolonged release formulation comprising:

(i) a polylactide polymer or poly(lactide-co-glycolide) co-polymer having an average molecular weight of from 5,000 Daltons to 50,000 Daltons;
(ii) a ratio of lactide to glycolide of between 100:0 to 50:50; and
(iii) from about 10 to 55% by weight of anastrozole based upon the total weight of the formulation;

wherein the prolonged release formulation is a microparticle formulation and wherein
(i) for anastrozole loadings at lactide to glycolide ratios given in the table below the average molecular weight of the polymer is above the figure given in the table:

| Lactide/Glycolide | Loading | Average Molecular Weight |
|---|---|---|
| 50:50- 65:35 | >=10% | >15,000 |
| 50:50- 65:35 | >20% | >25,000 |
| 65:35 | >30% | >45,000 |
| 66:34 - 75:25 | >20% | >15,000 |
| 66:34 - 75:25 | >30% | >40,000 |
| 76:24 - 85:15 | >20% | >10,000 |
| 76:24 - 85:15 | >30% | >30,000 |
| 85:15 | >40% | >45,000 |
| 86:14 - 95:5 | >20% | >10,000 |
| 86:14 - 95:5 | >30% | >15,000 |
| 86:14 - 95:5 | >35% | >30,000 |
| 86:14 - 95:5 | >40% | >40,000 |
| 96:4 - 100/0 | >30% | >17,000 |
| 96:4 - 100/0 | >40% | >35,000 |

and
(ii) for anastrozole loadings above 30% the lactide/glycolide ratio is 65:35 or above and for anastrozole loadings above 40% the lactide/glycolide ratio is 85:15 or above.

According to a further aspect of the present invention there is provided a prolonged release formulation comprising:

(i) a polylactide polymer or poly(lactide-co-glycolide) co-polymer having an average molecular weight of from 5,000 Daltons to 50,000 Daltons;
(ii) a ratio of lactide to glycolide of between 100:0 to 50:50; and
(iii) from about 10 to 55% by weight of anastrozole based upon the total weight of the formulation;

wherein the prolonged release formulation is a microparticle formulation and wherein
(i) for anastrozole loadings at lactide to glycolide ratios given in the table below the average molecular weight is above the figure given in the table:

| Lactide/Glycolide | Loading | Average Molecular Weight |
|---|---|---|
| 50:50 - 65:35 | >=10% | >15,000 |
| 50:50 - 65:35 | >20% | >25,000 |
| 66:34 - 75:25 | >20% | >15,000 |
| 76:24 - 85:15 | >20% | >10,000 |
| 86:14 - 95:5 | >30% | >15,000 |

and
(ii) for anastrozole loadings above 30% the lactide/glycolide ratio is 65:35 or above and for anastrozole loadings above 40% the lactide/glycolide ratio is 85:15 or above.

According to a further aspect of the present invention there is provided a prolonged release formulation comprising:

(i) a polylactide polymer or poly(lactide-co-glycolide) co-polymer having an average molecular weight of from 5,000 Daltons to 50,000 Daltons;
(ii) a ratio of lactide to glycolide of between 100:0 to 50:50; and
(iii) from about 10 to 55% by weight of anastrozole based upon the total weight of the formulation;

wherein the prolonged release formulation is a microparticle formulation and wherein the minimum average molecular weight of the polymer can be calculated from the equation below:

$$MW = -71.88 + 25.0(0.5 + \tanh(14.3(f_A - 0.33))) + 84.5(f_G + 1.08) - 12.8(f_G + 1.08)^2$$

wherein
$f_A$ is the fraction of anastrozole wherein 1.0 is 100%, i.e. 5% would be expressed as 0.05; and
$f_G$ is the fraction of glycolide wherein 1.0 is 100%, i.e. 50% would be expressed as 0.50.

Formulations of the invention include implants and microparticle formulations.

Microparticles are generally powders consisting substantially of solid spherical particles 2 millimeters or less in diameter, usually 500 microns or less in diameter. A microparticle has its active agent dispersed or dissolved throughout the particle; that is, the internal structure is a matrix of the agent and excipient, usually a polymeric excipient. Biodegradeable controlled-release microparticles, such as those based on polylactide or poly(lactide-co-glycolide) (PLGA), typically release their agent via a combination of drug diffusion and polymer erosion, which often gives multiphasic release i.e. comprising of an initial period where some drug is released, a second short period where less drug is released, and a third period during which most of the remainder of the drug is released. The rate of drug diffusion and polymer erosion can sometimes be controlled to get near zero-order profiles, by altering parameters such as the drug loading, ratio of lactide/glycolide used to prepare the copolymers, the molecular weight and crystallinity of the polymer or by using blends of polylactide and PLGA polymers [for more information the reader is referred to standard texts in the art such as: Biodegradables and delivery systems for contraception, edited by E.S.E Hafez and W.A.A van Os, MTP Press Ltd.,

Lancaster, UK 1980 or Controlled Release of Bioactive Agents from Lactide/Glycolide Polymers. D. H. Lewis. in: Biodegradable Polymers as Drug Delivery Systems, edited by M. Chasin and R. Langer, Marcel Dekker, Inc., New York 1990].

Hereinafter, the term microparticle will include microspheres, microcapsules and particles in general, with an internal structure comprising a matrix of agent and excipient.

A wide variety of methods to prepare microparticles are described in the literature. Several of these methods make use of emulsions to make microparticles, in particular to make microparticles less than 2 millimeters in diameter. To give a general example of such processes, one can dissolve a polymer in a suitable organic solvent (the polymer solvent), dissolve or disperse an agent in this polymer solution, disperse the resulting polymer/agent mixture into an aqueous phase (the processing medium) to obtain an oil-in-water emulsion with oil microdroplets dispersed in the processing medium, and remove the solvent from the microdroplets to form microparticles. These processes can also be performed with water-in-oil emulsions and with double emulsions, i.e. water-in-oil-in-water emulsions. The use of emulsion-based processes that follow this basic approach is described in several U.S. patents. For example, U.S. Pat. No. 4,384,975 describes the production of microparticles by forming an emulsion and then slowly removing the polymer solvent from the microdroplets in the emulsion by vacuum distillation. As another example, U.S. Pat. No. 3,891,570 discloses a method in which the polymer solvent is removed from the microdroplets in the emulsion by applying heat or reducing the pressure in the fabrication vessel. In still another example, U.S. Pat. No. 4,389,330, the polymer solvent is partially removed from the microdroplets in the emulsion by vacuum distillation (preferably 40 to 60% of the polymer solvent) and then the remainder of the polymer solvent is extracted to solidify the microparticles.

The main disadvantage of the above methods is that low molecular weight water-soluble compounds tend to partition out of the microdroplets and into the aqueous phase before the microparticles have hardened leading to low drug encapsulation efficiencies. An alternative approach is to use a solvent extraction method which involves rapidly extracting the polymer solvent from the microdroplets to harden microparticles quicker and thus reduce drug partitioning into the aqueous phase (see Example 3). Non-emulsion based methods, such as phase separation, spray drying and spray chilling can also be used to prepare microparticles [For information on such methods the reader is referred to standard texts in the art such as: Biodegradable Microspheres: Advances in Production Technology. J. Benoit and H. Marchais. In: Microencapsulation, Methods and Industrial Applications, edited by Simon Benita, Marcel Dekker, Inc., New York 1996]

The microparticles are administered in an appropriate vehicle. The vehicle may be sterile water, phosphate buffered saline, or other conventional vehicle for administering microparticles. Additives may be present to reduce agglomeration of the microparticles. Conveniently, mannitol may be present in about 4 to 7 weight % of the vehicle. Other physiologically acceptable additives may include non-ionic detergents, e.g. Tween, polysorbate etc., which if present, would be present in from about 0.05 to 0.2% weight of the vehicle, viscosity enhancing agents, e.g. carboxymethylcellulose, in the range of about 0.1 to 1% weight of the vehicle and other conventional additives, as appropriate. The microparticles are dispersed in the vehicle immediately before use.

Implants, such as monolithic implants, are solid structures in which an agent is dissolved or dispersed throughout the matrix; that is, the internal structure is a matrix of the agent and excipient. Such implants have a unitary structure, typically having dimensions greater than about 0.5mm, more preferably from 1mm to 30mm and typically have a diameter to facilitate delivery via a 14 or 16 gauge needle. The geometry and size of the implant will depend upon the method used to prepare the implant, the means used to insert the implant in a patient, the composition of the formulation and the dose and release profile of anastrozole required. Insertion of the implant can be through an incision or a large bore needle, depending on the size of the implant. Suitable geometries include slabs, discs cylinders, rods, spheres or pellets. The geometry of a cylindrical rod provides a convenient geometry for sub-dermal implantation into a patient using a needle or conventional surgical instruments such as a trochar.

Biodegradeable controlled-release monolithic implants, such as those based on polylactide or poly(lactide-co-glycolide) (PLGA), typically release their agent via a combination of drug diffusion and polymer erosion, which often gives multiphasic release i.e. comprising of an initial period where some drug is released, a second short period where less drug is released, and a third period during which most of the remainder of the drug is released. The rate of drug diffusion and polymer erosion can sometimes be controlled to get near zero-order profiles, by altering parameters such as the drug loading, ratio of lactide/glycolide used to prepare the copolymers, the molecular weight and crystallinity of the polymer or by using blends of polylactide and PLGA polymers [for more information the reader is referred to standard texts as referred under microparticles above].

Implants can be manufactured by standard methods such as compression moulding, injection moulding and screw extrusion [For information on such methods the reader is referred to standard texts in the art such as: Controlled Release of Bioactive Agents from Lactide/Glycolide Polymers. D. H. Lewis. In: Biodegradable Polymers as Drug Delivery Systems, edited by M. Chasin and R. Langer, Marcel Dekker, Inc., New York 1990].

Implants can also be pulverised into powders, to form microparticles, by grinding or milling under cooling with dry ice or liquid nitrogen so that the preparation is kept at a temperature of about -10°C to about -100°C, or by any conventional pulverisation method at room temperature or lower temperature. The pulverised material, having an injectable particle size (e.g. 0.1-000μm), can be suspended in a suitable vehicle for injection.

The term 'about' when relating to the proportion of anastrozole in the formulation refers to ± 5% weight percent of the formulation, particularly ± 2% weight percent of the formulation.

The term 'about' when relating to the duration of release of anastrozole from the formulation refers to ± 2 days, particularly ± 1 day, further particularly ± 12 hours.

The term 'about' when relating to the molecular weight of the polymer refers to ± 5 kDa, particularly 2 kDa, further particularly ± 1 kDa.

The term "aqueous physiological environment" as used herein refers to the body of a warm blooded animal, particularly man, and especially the subcutaneous environment of such a body. These conditions may be simulated *in vitro* by placing a formulation in an aqueous dissolution medium, optionally buffered to a physiological pH, at a temperature of from 35 to 40°C. A suitable dissolution medium comprises a saline solution buffered to a pH of approximately 7.4 using a phosphate buffer, for example phosphate buffered saline or McIlvaines citric acid phosphate. Preferably, the aqueous dissolution medium is maintained at a temperature of 37C ± 2°C. The amount of anastrozole released over a given time period may be determined by sampling the dissolution medium and measuring the concentration of anastrozole using a suitable analytical method, for example HPLC.

The term "continuous" as used herein refers to a continual release of anastrozole from the implant or microparticle formulation for at least 10 days after implantation or injection into an aqueous physiological environment. The rate of release of the anastrozole may vary during the at least 10 day period, for example a short "initial burst" of anastrozole may be observed shortly after implantation/injection followed by a period of lower release. However, there are no periods in the at least 10 days following implantation or injection where the release of anastrozole from the implant or microparticle formulation is insufficient to maintain *in vivo* levels of anastrozole. Preferred levels of release of anastrozole include at least 0.25mg per day, particularly at least 0.5mg per day, more particularly about 1mg of the anastrozole per day when the implant or microparticle formulation is placed in an aqueous physiological environment. Preferably, the rate of release of the anastrozole is approximately constant, but always continuous, over most of the at least 10 day period.

The term 'kDa' refers to kilodaltons.

Formulations of the invention comprise polymers of lactic acid and glycolic acid.

The polylactide polymer is a homopolymer wherein all the repeat units of the polymer are of the Formula (1):

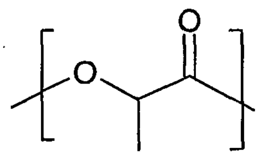

Formula (1)

The repeat units are selected from polymers in the D- configuration or a mixture of the Land D- configurations. Preferably the repeat units of Formula (1) comprise a mixture of L- and D- configurations. When the polymer comprises a mixture of repeat units in the Land D- configurations the ratio of L- to D- units in the polymer is preferably from 25:75 to 75:25, more preferably from 30:70 to 70:30 and especially approximately 1:1.

Each polymer chain of the polylactide or poly(lactide-co-glycolide) co-polymer is preferably terminated by one hydroxy group and one -COOH group. However, in embodiments of the present invention other terminal groups may be present, provided that the presence of such terminal groups do not adversely affect the release of anastrozole from the formulation. Suitable terminal groups other than -OH or -COOH which may be present on the polymer include esters formed by reacting an appropriate acid or alcohol with the -OH and/or -COOH end group(s) of the polymer. Suitable esters include alkyl (preferably $C_{1-4}$-alkyl) or aralkyl (preferably benzyl) esters.

The polylactide polymer may comprise a single polylactide homo polymer or a blend of two or more polylactide homo polymers. A blend of two or more polylactide polymers, or, two or more poly(lactide-co-glycolide) polymers or blends of polylactide polymers and poly(lactide-co-glycolide) can be used to provide further control over the rate of release of anastrozole from the formulation, thereby providing a more consistent rate of release over the life-time of the implant in a physiological type environment. Blends of polymers can also be used for minimising "flat spots" in the anastrozole release profile, thereby providing a smooth, steady release of the anastrozole from the formulation.

The polylactide or poly(lactide-co-glycolide) polymer may be prepared using known methods. A preferred method for the preparation of polylactide and poly(lactide-co-glycolide) polymers is ring-opening polymerisation of heterocyclic monomers composed of two lactic or two glycolic acid units, namely, lactide and glycolide, respectively. The ring opening polymerisation is performed under conditions of elevated temperature and in the presence of a suitable

catalyst using conditions well known in the polymer art.

Suitable catalysts for the ring-opening polymerisation include, but are not limited to, zinc, zinc oxide, zinc chloride, p-toluene sulphonic acid, antimony catalysts, for example antinomy trifluoride, or organo-tin catalysts, for example stannous octoate (stannous 2-ethylhexanoate) or tin chloride.

A suitable reaction temperature for the ring-opening polymerisation is from about 120°C to about 240°C, more preferably from 140°C to 200°C. The ring opening polymerisation is preferably performed over a period of from 1 to 10 hours more preferably from 2 to 6 hours.

Preferably the ring opening polymerisation reaction is performed in the presence of a suitable chain termination agent thereby controlling the MW of the resultant polylactide or poly(lactide-co-glycolide) polymer. Suitable chain termination agents include water, a hydroxy-carboxylic acid such as lactic acid or an alcohol, such as a $C_{1-6}$-alkanol. [For further information on suitable methods the reader is referred to standard texts in the art such as: Biodegradable Polymers: Polyesters. S. Li and M. Vert. In: Encyclopedia or Controlled Drug Delivery Volume 1, edited by E Mathiowitz, Wiley-Interscience, New York 1999 or, Polylactic and Polyglycolic Acids as Drug Delivery Carriers. L. Brannon-Peppas and M. Vert. In: Handbook of Pharmaceutical Controlled Release Technology, edited by Donald L. Wise, Marcel Dekker, Inc., New York, 2000]

The methods used to prepare polylactide and poly(lactide-co-glycolide) polymers typically result in a mixture of individual polylactide polymer chains, many of which are of differing chain lengths. The polydispersity of a polymer provides an indication of the spread of chain lengths in such a mixture and is defined to be the ratio of the weight average molecular weight (MW) to the number average molecular weight ($M_n$). Suitably, the polydispersity of the polymer is from 1.3 to 4.5.

Particular novel formulations of the invention include, for example, formulations wherein the characteristics comprise any of the meanings defined hereinafter:-

(a) The formulation is a solid implant, such as a monolithic implant formulation;

(b) The formulation is a monolithic implant formulation typically having dimensions greater than about 0.5mm, particularly from 1mm to 30mm, with a diameter such that the implant can be delivered using a 14 or 16 gauge needle.

(c) The formulation is a monolithic implant formulation and is a slab, disc, cylinder, rod, sphere or pellet, particularly a cylindrical rod;

(d) The formulation is a microparticle formulation;

(e) The formulation is a microparticle formulation with an average diameter of less than 500 microns, particularly below 300 microns in diameter, more particularly below 200 microns in diameter.

(f) The weight average molecular weight of the polylactide polymer is between:

(i) 9,000 to 44,000 Daltons;
(ii) 11,000 to 44,000 Daltons; or
(iii) 21,000 to 31,000 Daltons

The weight average molecular weight of the poly(lactide-co-glycolide) polymer is between:

(i) 9,000 to 44,000 Daltons;
(ii) 11,000 to 44,000 Daltons; or
(iii) 21,000 to 31,000 Daltons

The weight average molecular weight (MW) of the polymer is measured using size exclusion chromatography (SEC) using polymer solutions in tetrahydrofuran (THF) with 2 x 30$cm^2$ mixed bed 'D's PLGel columns (supplier Polymer Laboratories) which have a linear range of MW 200-400 kDa; wherein the system is first calibrated using PL Easical PS-2 polystyrene calibrants with MW's in the range 580 to 400 kDa. The PLGel packing material is a highly cross linked spherical polystyrene/divinylbenzene matrix. A Wyatt Optilab DSP refractometer may be used for detection.

(g) The formulation comprises the following proportions of anastrozole:

(i) about 5 to 55% by weight;
(ii) about 10 to 55% by weight;
(iii) about 15 to 50% by weight;
(iv) about 20 to 40% by weight; or
(v) about 25% by weight;

(h) The formulation is a monolithic implant and comprises the following proportions of anastrozole:

(i) about 10 to 65% by weight;
(ii) about 20 to 65% by weight;
(iii) about 20% by weight;
(iv) about 30% by weight;
(v) about 40% by weight;
(vi) about 50% by weight; or
(vii) about 55% by weight;

(i) The formulation is a microparticle formulation and comprises the following proportions of anastrozole:

(i) about 5 to 55% by weight;
(ii) about 10 to 50% by weight;
(iii) about 10 to 30% by weight;
(iv) about 10% by weight;
(v) about 20% by weight; or
(vi) about 30% by weight;

(j) The duration of release is:

(i) At least about 7 days;
(ii) At least about 10 days;
(iii) At least about 14 days;
(iv) At least about 18 days;
(v) At least about 21 days;
(vi) At least about 25 days;
(vii) At least about 30 days;
(viii) At least about 40 days;
(ix) At least about 50 days wherein the formulation is a microparticle formulation, the molar ratio of lactide to glycolide is between 100:0 to 95:5, the polylactide polymer or poly(lactide-co-glycolide) co-polymer has a weight average molecular weight of from 15,000 Daltons to 20,000 Daltons, and the drug loading is between 8 and 17% by weight;
(x) At least about 60 days wherein the formulation is a microparticle formulation, the molar ratio of lactide to glycolide is between 100:0 to 95:5, the polylactide polymer or poly(lactide-co-glycolide) co-polymer has a weight average molecular weight of from 20,000 Daltons to 25,000 Daltons, and the drug loading is between 10 and 20% by weight;
(xi) At least about 70 days wherein the the formulation is a microparticle formulation, molar ratio of lactide to glycolide is between 100:0 to 95:5, the polylactide polymer or poly(lactide-co-glycolide) co-polymer has a weight average molecular weight of from 27,000 Daltons to 35,000 Daltons, and the drug loading is between 12 and 23% by weight;
(xii) At least about 80 days wherein the the formulation is a microparticle formulation, molar ratio of lactide to glycolide is between 100:0 to 95:5, the polylactide polymer or poly(lactide-co-glycolide) co-polymer has a weight average molecular weight of from 35,000 Daltons to 40,000 Daltons, and the drug loading is between 13 and 26% by weight; or
(xiii) At least about 90 days wherein the formulation is a microparticle formulation, the molar ratio of lactide to glycolide is between 100:0 to 95:5, the polylactide polymer or poly(lactide-co-glycolide) co-polymer has a weight average molecular weight of from 40,000 Daltons to 50,000 Daltons, and the drug loading is between 15 and 30% by weight;

(k) The formulation is a monolithic implant with the following characteristics:

| Lactide:Glycolide ratio | Molecular Weight (about, kDa) | Loading (about, % by weight) | Duration of release (at least, about) |
|---|---|---|---|
| 50:50 | ≥18-20 | 20% | 14 |
| 65:35 | ≥15 | 30-60% | 14 |
| 75:25 | ≥15 | 30-60% | 14 |

(continued)

| Lactide:Glycolide ratio | Molecular Weight (about, kDa) | Loading (about, % by weight) | Duration of release (at least, about) |
|---|---|---|---|
| 85:15 | ≥5-10 | 30-60% | 14 |
| 95:5 | ≥5-10 | 30-60% | 14 |
| D/L 100:0 | ≥5 | 30-60% | 14 |

(l) The formulation is a microparticle with the following characteristics:

| Lactide:Glycolide ratio | Molecular Weight (about, kDa) | Loading (about, % by weight) | Duration of release (at least, about) |
|---|---|---|---|
| 50:50 | ≥35-45 | 20-25% | 14 |
| 65:35 | ≥30-40 | 20-25% | 14 |
| 75:25 | ≥25-30 | 20-25% | 14 |
| 85:15 | ≥20 | 20-25% | 14 |
| 95:5 | ≥10 | 20-25% | 14 |
| D/L 100:0 | ≥10 | 20-25% | 14 |
| 50:50 | ≥50 | 30% | 14 |
| 65:35 | ≥40-45 | 30% | 14 |
| 75:25 | ≥35-40 | 30% | 14 |
| 85:15 | ≥30 | 30% | 14 |
| 95:5 | ≥25 | 30% | 14 |
| D/L 100:1 | ≥17 | 30% | 14 |
| 95:5 | ≥45-50 | 40% | 14 |
| D/L 100:0 | ≥40 | 40% | 14 |

Durations of release in sections k and 1 above relate to the duration of release when measured in an *in vitro* system as described in Example 1 below. Generally *in vivo,* longer durations of release than *in vitro* were observed, for example see the following table:

| | Lactide: Glycolide ratio | Molecular Weight (kDa) | Loading (% by weight) | Release Duration (Days) (in-vitro) | Release Duration (Days) (in-vivo) |
|---|---|---|---|---|---|
| Monolithic Implant | 95:5 | 26 | 40% | 30 | 45-50 |
| Monolithic Implant | 95:5 | 26 | 60% | 30 | 45-50 |
| Microparticle | 95:5 | 26 | 8.2% | 35 | 55 |

According to a further aspect of the present invention there is provided a medicament comprising a formulation of the present invention.

The use of a combination of two or more different implant or microparticle formulations according to the present invention enables a wide range of release profiles to be achieved by appropriate selection of polymers and/or loading of anastrozole. This may be advantageous for the treatment of certain diseases. For example, it may be desirable to provide a high initial dose of anastrozole, followed by a lower dose for the remainder of the treatment. This may be achieved by selecting a first implant or microparticle formulation which has a high initial release rate of anastrozole and

a second implant or microparticle formulation which has a more constant release rate. The cumulative anastrozole release from the two formulations thereby providing a high initial dose followed by a substantially constant release rate for the remainder of the treatment period. Alternatively, by appropriate selection of two or more different implant or microparticle formulations it is possible to provide a cumulative release of anastrozole which is substantially zero order (i.e. substantially constant) throughout the treatment period.

The release profile of anastrozole from the first and second implants/microparticle formulations may be controlled by, for example, varying the lactide:glycolide ratio and/or the molecular weight of the polylactide or poly(lactide-co-glycolide) and/or the loading of anastrozole in the implant.

The first and second implants or microparticle formulations may be implanted or injected (preferably subcutaneously) at the same or different parts of a patients body.

Thus, according to a further aspect of the invention there is provided a prolonged release formulation comprising a combination of two or more different implants or microparticles of the invention.

According to a further aspect of the present invention there is provided a process for the preparation of a monolithic implant formulation according to the present invention comprising the steps:

(i) preparing a solution or dispersion of anastrozole in a solution comprising the polylactide or poly(lactide-co-glycolide) polymer and a suitable organic solvent;
(ii) removing substantially all of the organic solvent from the solution or dispersion formed in step (i); and
(iii) forming the product of step (ii) into a monolithic implant of the required dimensions.

*Process step (i)*

Preferably anastrozole and polymer are dissolved in an organic solvent solution. A suitable organic solvent includes for example, glacial acetic acid. Typically anastrozole and the polymer are dissolved by stirring at room temperature.

*Process step (ii)*

The organic solvent may be removed in step (ii) using any convenient technique, for example by evaporation or freeze-drying. Preferably at least 80% by weight, more preferably at least 90% by weight of the organic solvent is removed in step (ii) of the process. A preferred method for removing organic solvent in step (ii) is to add the polymer/solvent/anastrozole mixture drop-wise into liquid nitrogen and remove the organic solvent from the frozen droplets by freeze-drying. Optionally further organic solvent may be removed from the resulting freeze dried material by, for example, vacuum drying or oven drying. Suitably the temperature during the vacuum or oven drying is from 20°C to 65°C, for example from 25 to 60°C.

*Process step (iii)*

The polymer/anastrozole composition may be formed into a monolithic implant by, for example compression moulding, injection moulding or screw extrusion.

According to a further aspect of the present invention there is provided a process for the preparation of a monolithic implant formulation of according to the present invention comprising the steps:

(i) preparing a mixture of anastrozole and the polylactide or poly(lactide-co-glycolide) polymer;
(ii) forming the mixture into a monolithic implant of the required dimensions.

*Process step (i)*

Anasrozole and polymer may be blended by any convenient method available to the skilled man, such as a mortar and pestle.

*Process step (ii)*

The polymer/anastrozole composition may be formed into a monolithic implant by, for example compression moulding, injection moulding or screw extrusion.

According to a further aspect of the present invention there is provided a process for the preparation of a microparticle formulation according to the present invention comprising the steps:

(i) preparing a solution or dispersion of anastrozole in a solution comprising the polylactide or poly(lactide-co-glycolide) polymer and a suitable organic solvent;
(ii) dispersing the solution prepared in (i) into an aqueous processing medium to form an oil-in-water emulsion;
(iii) removing substantially all of the organic solvent from the emulsion formed in step (ii) to form microparticles; and
(iv) washing and drying the microparticles.

*Process step (i)*

Preferably anastrozole and polymer are dissolved in an organic solvent solution. Suitable organic solvents include dichloromethane, ethyl acetate and methyl formate or mixtures thereof. Preferably the solution contains from about 5 weight percent to about 35 weight percent of polymer and anastrozole of which from about 10 weight percent to about 60 weight percent of the polymer/anastrozole component is anastrozole. Typically anastrozole and the polymer are dissolved by stirring at room temperature.

*Process step (ii)*

The emulsification step is typically performed by adding a small volume of the polymer/anastrozole solution to a larger volume of aqueous processing medium containing a surfactant, which is included to allow formation of a stable emulsion and prevent agglomeration. The process medium is mechanically agitated with devices such as homogenisers, propellers, or the like as the polymer/anastrozole solution is added to the processing medium. Typically the ratio of the smaller volume to the larger volume is about 1:100. Examples of compounds that can be used as surfactants include methyl cellulose, poly(vinyl alcohol) (PVA), poly(vinyl pyrrolidone) and gelatin, preferably methyl cellulose. The concentration of surfactant in the process medium should be sufficient to stabilize the emulsion.

*Process step (iii)*

Following formation of the emulsion, the processing medium containing the microdroplets is continually agitated/stirred to allow the organic solvent to partition from the organic microdroplets into the aqueous processing medium and subsequently evaporate to leave behind hardened microparticles. Typically this takes 2-12 hours depending upon the solvent used, the volume ratio of solvent/processing medium and the method of agitation of the processing medium. Optionally, as soon as the emulsion forms, all of the processing medium containing the organic microdroplets is immediately transferred to a yet larger volume of aqueous extraction medium to rapidly extract the organic solvent from the microdroplets and further aid formation of the microparticles. The extraction medium, which is typically water, is also mechanically agitated with devices such as homogenisers, propellers, or the like as the processing medium containing the organic microdroplets is added to the extraction medium. Typically this takes 15 to 60 minutes depending upon the solvent used, the volume ratio of solvent/processing medium and the method of agitation of the extraction medium. Typically the ratio of the larger processing volume to the yet larger extraction volume is at least about 1:3.

*Process step (iv)*

After extraction of all or almost all of the solvent from the microdroplets, the hardened microparticles are collected by centrifugation or filtration, or the like and washed with distilled water before finally being dried by lyophilisation, or the like.

According to a further aspect the formulations of the present invention are provided for use in a method for treating a warm blooded animal (preferably a human) suffering from a condition treatable by anastrozole comprising administering thereto a formulation according to the present invention.

According to a further aspect of the present invention there is provided the use of a formulation of the present invention in the manufacture of a medicament for the treatment of a condition treatable by anastrozole (preferably breast cancer) in a warm blooded animal (preferably a human).

According to a further aspect the formulations of the present invention are provided for use in a method for treating a warm blooded animal (preferably a human) suffering from a condition treatable by anastrozole (preferably breast cancer) comprising administering thereto a monolithic implant according to the present invention.

According to a further aspect of the present invention there is provided the use of a monolithic implant of the present invention in the manufacture of a medicament for the treatment of a condition treatable by anastrozole (preferably breast cancer) in a warm blooded animal (preferably a human).

According to a further aspect the formulations of the present invention are provided for use in a method for treating a warm blooded animal (preferably a human) suffering from a condition treatable by anastrozole (preferably breast cancer) comprising administering thereto a microparticle formulation according to the present invention.

According to a further aspect of the present invention there is provided the use of a microparticle formulation of the present invention in the manufacture of a medicament for the treatment of a condition treatable by anastrozole (preferably breast cancer) in a warm blooded animal (preferably a human).

According to a further aspect of the present invention there is provided a formulation according to the present invention for use as a medicament in the treatment of a condition treatable with anastrozole (preferably breast cancer).

According to a further aspect of the present invention there is provided a monolithic implant according to the present invention for use as a medicament in the treatment of a condition treatable with anastrozole (preferably breast cancer).

According to a further aspect of the present invention there is provided a microparticle formulation according to the present invention for use as a medicament in the treatment of a condition treatable with anastrozole (preferably breast cancer).

The formulations according to the present invention are useful in the treatment of a wide variety of medical conditions requiring the administration of an aromatase inhibitor, such as anastrozole. Such medical conditions include, but are not limited to, hormone dependent diseases such as breast cancer, ovarian cancer, endometriosis and benign prostatic hypertrophy.

The dose of anastrozole required for the treatment of a particular condition will be dependent upon both the condition being treated and the animal to which it is administered. For example, for the treatment and prevention of breast cancer, the dose of anastrozole is generally 1mg per day.

According to a further aspect the formulations of the present invention, are provided for use in a method for administering anastrozole to a warn blooded animal, especially a human, comprising injecting (preferably subcutaneously) a formulation according to the present invention in the warm blooded animal.

According to a further aspect the formulations of the present invention, are provided for use in a method for administering anastrozole to a warm blooded animal, especially a human, comprising injecting (preferably subcutaneously) a microparticle formulation according to the present invention in the warm blooded animal.

According to a further aspect the formulations of the present invention, are provided for use in a method for administering anastrozole to a warm blooded animal, especially a human, comprising implanting (preferably subcutaneously) a monolithic implant according to the present invention in the warm blooded animal.

The monolithic implant may be implanted using conventional medical techniques. For example, subcutaneous implantation may be achieved using a needle or via a trochar or the like.

The invention is further illustrated by the following examples wherein all parts are by weight unless otherwise stated.

Poly(lactide-co-glycolide) and Polylactide Polymers

The Poly(d1-lactide-co-glycolide) (PLGA) and Poly(dl-lactide) (PLA) polymers shown in Table 1 were prepared via ring opening condensation of d1-lactide and glycolide dimmers. Polymers A-F are all uncapped with the terminal residues existing as carboxylic acids. Polymer G (100 DL 2M, supplied by Alkermes, Wilmington, USA) is end-capped with terminal residues functionalised with methyl esters.

**Table 1**

| Polymer | dl-Lactide/Glycolide molar proportion | MW (kDa) |
|---|---|---|
| Polymer A | 50/50 | 18 |
| Polymer B | 75/25 | 20 |
| Polymer C | 85/15 | 23 |
| Polymer D | 95/5 | 26 |
| Polymer E | 100/0 | 9 |
| Polymer F | 100/0 | 44 |
| Polymer G | 100/0 (methyl ester end capped) | 15.7 |

**Example 1: Monolithic implants**

The implants shown in Table 2 were prepared by melt extrusion of mixtures of drug and polymer. Batch sizes of up to 1 gram were prepared by weighing the amounts shown in Table 2 of anastrozole and the relevant polymer from Table 1 into a mortar and blending together with the aid of a pestle until a visually homogenous mix was obtained. Drug/polymer blends prepared in this manner were extruded using a three piece, one gram capacity, stainless steel extruder consisting of a barrel/nozzle, base and die with a diameter of 1.1mm. The extruder was heated using a pipe clamp, the temperature of which was controlled through a feedback circuit containing a thermocouple, located in thermal contact with the extruder barrel, and a custom built controller unit. Pressure was applied to the extruder die via a standard hydraulic KBr Press (Specac). Extrusion pressure was controlled manually. All extrudates were manufactured using the same nominal heating programme. The temperature of the extruder was initially raised to a nominal value of 85°C over a period of 30-60 minutes, and then held at this temperature for a further 15 minutes. Extrusion of the drug/polymer melt was then performed by applying pressures of up to 1 Ton (nominal) to the extruder die. The resulting extrudates were cut into the required lengths.

In order to determine drug content, 10mg of implant was dissolved in 1ml of acetic acid (Analytical grade, BDH Chemicals, UK) in a 25ml volumetric flask. Following complete dissolution, the solution was made up to volume with MilliQ water, causing the polymer to precipitate. The solid was then filtered off using a 0.45 μm Millex PTFE filter (Millipore, UK) and an aliquot of the filtrate was assayed using an isocratic HPLC method (HiChrom RPB column, mobile phase 70/30 v/v mixture of 0.1% v/v Trifluoracetic acid in water and Acetonitrile). Anastrozole concentrations were calculated on the basis of peak area, against external calibration standards, prepared by dissolving weighed quantities of anastrozole in pure water. Drug content analysis was performed in duplicate for each batch of implants and drug loadings shown in Table 2 represent the mean of two determinations.

Dissolution tests were performed on three samples of each implant formulation, the weights of which are shown in Table 2. All release profiles shown represent the mean of 3 dissolution trials, run in parallel.

**Table 2**

| Implant | Polymer from Table 1 | Polymer weight (g) | Anastrozole weight (g) & determined drug loading (% w/w) | Implant weight (mg) |
|---|---|---|---|---|
| Implant A | Polymer A | 0.8 | 0.2, 20.0% loading | 10.33, 9.52, 9.81 |
| Implant B | Polymer D | 0.6 | 0.4, 36.9% loading | 6.52, 4.12, 5.55 |
| Implant C | Polymer D | 0.4 | 0.6, 60.8% loading | 5.52, 3.04, 4.18 |
| Implant D | Polymer E | 0.6 | 0.4, 39.6% loading | 5.14,4.93, 4.68 |

<u>Release Profiles</u>

The rate of release of the anastrozole *in vitro* was measured by placing the implants shown in Table 2 in 10ml of phosphate buffered saline (PBS) comprising 1.38g $Na_2HPO_4$, 0.19g $KH_2PO_4$, 8.00g NaCl and 0.20 g $NaN_3$ per litre of deionised water (adjusted to pH 7.4 +/-0.05 using 0.1M HCl) and storing in an incubator at a temperature of 37°C. At regular time intervals, 1ml samples of dissolution medium were withdrawn from each sample vial. An equal volume of fresh PBS was immediately returned to each vial after sampling. Sampling was performed manually with a pipette. The anastrozole content of all dissolution media samples were measured using an isocratic HPLC method (HiChrom RPB column, mobile phase 70/30 v/v mixture of 0.1% v/v Trifluoracetic acid in water and Acetonitrile). Anastrozole concentrations were calculated on the basis of peak area, against external calibration standards, prepared by dissolving weighed quantities of anastrozole in pure water. The release profiles, calculated from the measured anastrozole concentrations are shown in Figure 1.

Figure 1 shows that Implant A provided continual release of anastrozole over a period of 2 weeks with minimal drug burst over the first 24 hours. Implants B and C, prepared from a polymer with higher lactide content (PLGA 95/5, Lactide/Glycolide) and molecular weight (26 kDa) at 40 and 60%w/w drug loading respectively, exhibited continual release for over 25 days. Implant D, prepared from a dl-polylactide with 9 kDa molecular weight was also able to provide controlled release for a duration of 20 days at 40% w/w drug loading. These results show that implants capable of releasing anastrozole in a controlled manner over an extended period of time can be formulated at high drug loadings using a range of PLGA and PLA polymers.

**Example 2: Microparticles prepared by solvent evaporation**

Microparticles were prepared using a single emulsion technique. A 4ml solution containing 0.4g of anastrozole and polymer at the desired weight ratio (as detailed in Table 3.) in dichloromethane (DCM) was emulsified at high speed (2100 rpm) with an overhead stirrer and stainless steel straight blade impeller (Heidolph, Type RZR1 Germany) for 3 minutes with 360ml of 0.25%w/v methylcellulose (Methocel MC, Fluka Chemicals, UK) solution. The resulting o/w emulsion was stirred for a further 30 minutes at 800rpm to allow the solvent to evaporate. Finally, the resultant microparticles were cleaned by sedimentation and resuspension in chilled distilled water a total of three times, lyophilised and stored at 2-8°C.

In order to determine drug content, 10mg of microparticles were dissolved in 1ml of acetic acid (Analytical grade, BDH Chemicals) in a 25ml volumetric flask. Following complete dissolution, the solution was made up to volume with MilliQ water, causing the polymer to precipitate. The solid was filtered off using a 0.45 μm Millex PTFE filter (Millipore, UK) and an aliquot of the filtrate was assayed using an isocratic HPLC method (HiChrom RPB column, mobile phase 70/30 v/v mixture of 0.1% v/v Trifluoracetic acid in water and Acetonitrile). Anastrozole concentrations were calculated on the basis of peak area, against external calibration standards, prepared by dissolving weighed quantities of anastrozole in pure water. Drug content analysis was performed in duplicate for each batch of microparticles and drug loadings shown in Table 2 represent the mean of two determinations.

Anastrozole loadings of up to 11 % w/w were achieved but loading efficiencies were only 30-36%. These modest loading efficiencies appear to be due to the aqueous solubility of anastrozole (0.53mg/ml) causing the drug to simply diffuse into the aqueous phase during the microparticle hardening phase. Increasing the PLGA and drug content of the dispersed phase from 100mg/ml through to 400mg/ml increased core loading efficiencies to 48%. Substituting DCM for ethyl acetate (EA) or methyl formate (MF) also increased loading efficiency, however batches produced using either of the two solvents were found to contain a large proportion of non-spherical material. Improvement in morphology was achieved by adding a small amount of the solvent to the external phase prior to the emulsification step but encapsulation efficiency was compromised as a result. The use of a mixed solvent system (EA:DCM 3:1) as the dispersed phase increased loading efficiency to 83% and gave particles with spherical morphology.

**Table 3.**

| Aqueous phase | Internal phase | Polymer weight (g) | Anastrozole weight (g) & theoretical drug loading (% w/w) | Determined drug loading (% w/w) | Drug loading efficiency (%) | Yield % |
|---|---|---|---|---|---|---|
| 0.25% Methocel | DCM | 0.36 | 0.04, 10% loading | 3.6 | 35.7 | 50-60 |
| 0.25% Methocel | DCM | 0.26 | 0.14, 35% loading | 10.6 | 30.2 | 27.4 |
| 0.25% Methocel | DCM | 0.70 | 0.10, 12% loading | 4.9 | 40.7 | 51.0 |
| 0.25% Methocel | DCM | 1.41 | 0.19, 12% loading | 5.7 | 47.5 | 86.4 |
| 0.25% Methocel | EA | 0.35 | 0.05, 12% loading | 10.8 | 90.0 | 43.5 |
| 0.25% Methocel | MF | 0.35 | 0.05, 12% loading | 12.0 | 100.0 | 58.5 |
| 0.25% Methocel + 10% v/v MF | MF | 0.70 | 0.10, 12% loading | 3.0 | 24.6 | 68.2 |
| 0.25% Methocel + 5% v/v EA | EA | 0.70 | 0.10, 12% loading | 2.0 | 16.3 | 60.0 |
| 0.25% Methocel | EA: DCM (3: 1) | 0.70 | 0.10, 12% loading | 10.0 | 83.0 | 78.5 |

**Example 3: Microparticles prepared by solvent extraction**

The microparticle formulations shown in Table 4 were prepared by a single emulsion technique, using an extraction process. A 4ml solution containing 1.6g of anastrozole and polymer at the desired weight ratio (as detailed in Table 4.) in DCM was emulsified at high speed (2100 rpm) with an overhead stirrer and stainless steel straight blade

impeller (Heidolph, Type RZR1 Germany) for 3 minutes with 360ml of 0.25%w/v methylcellulose (Methocel MC, Fluka Chemicals) solution. Following formation of the o/w emulsion, the entire contents of the vessel was immediately poured into 1.5L of ultrapure water stirring at around 200-400rpm to rapidly extract the organic solvent out of the emulsion droplets. The resultant microparticles were allowed to harden in the water for 1 hour before being cleaned by sedimentation and resuspension in chilled distilled water a total of three times. Finally, the microparticles were lyophilised and stored at 2-8°C.

The microencapsulation of anastrozole by solvent extraction was investigated to achieve greater loading efficiencies. Microparticles with anastrozole contents of 23-25% (32% theoretical) were prepared with an average of 72.6% drug loading efficiency. SEM analysis showed the micropsheres to be smooth, solid spheres.

**Table 4**

| Microparticle formulation | Polymer from Table 1 | Polymer weight (g) | Anastrozole weight (g) & determined drug loading (% w/w) | Drug loading efficiency (%) | Microparticle weight (mg) |
|---|---|---|---|---|---|
| Formulation A | Polymer B | 1.09 | 0.51, 22.8% loading | 71.3 | 7.51,4.60, 4.87 |
| Formulation B | Polymer C | 1.08 | 0.52, 23.3% loading | 72.8 | 8.19,5.33, 6.66 |
| Formulation C | Polymer D | 1.08 | 0.51, 24.5% loading | 64.5 | 9.90, 10.87, 10.40 |
| Formulation D | Polymer F | 1.09 | 0.52, 24.0% loading | 75.0 | 5.92, 10.79, 6.94 |
| Formulation E | Polymer G | 1.08 | 0.51, 25.3% loading | 79.2 | 8.44, 7.36, 6.80 |

The release profile of anastrozole from each batch of microparticles shown in Table 4. was measured *in vitro* as described in Example 1. The release profiles are shown in Figure 2. In general, 10-20% of the anastrozole was released within the first 24 hours followed by relatively constant release for durations ranging from 14 to 40 days.

Brief Description of the Drawings

Figure 1 shows the *in vitro* release of anastrozole (drug) from monolithic implants comprising a poly(DL-lactide-co-glycolide) co-polymer (PLGA) with a composition [50/50 L/G wt%] and weight average molecular weight (MW) of 18 kDa (black squares as data points) and a drug loading of 20% w/w, **"implant A";** a PLGA with a composition [95/5 L/G wt%] and MW of 26 kDa and a drug loading of 36.9%w/w (black diamonds as data points), **"implant B"** and 60.8% w/w (grey triangles as data points), **"implant C";** and a D/L-PLA polymer with MW of 9 kDa with a 39.6% w/w loading of anastrozole (grey circles as data points), **"implant D",** wherein the x axis is time in days and the y axis is percentage of anastrozole released.

Figure 2 shows the *in vitro* release of anastrozole from microparticle formulations comprising various co-polymers with different lactide contents and molecular weights according to the present invention. Curve A (black squares as data points) corresponds to a PLGA with a composition [75/25 L/G wt %] having a MW of 20kDa and a drug loading of 22.8% w/w, **"Formulation A".** Curve B (black diamonds as data points) corresponds to a PLGA with a composition [85/15 L/G wt %] having a MW of 23kDa and a drug loading of 23.3% w/w, **"Formulation B".** Curve C (grey triangles as data points) corresponds to a PLGA with a composition [95/5 L/G wt %] having a MW of 26kDa and a drug loading of 24.5% w/w, **"Formulation C".** Curve D (grey circles as data points) corresponds to a D/L-PLA with a MW of 44kDa and a drug loading of 24.0% w/w, **"Formulation D".** Curve E (white circles as data points) corresponds to a methyl ester end capped D/L-PLA with a MW of 15.7kDa and a drug loading of 25.3% w/w, **"Formulation E" ,** wherein the x axis is time in days and the y axis is percentage of anastrozole released.

**Claims**

1. A prolonged release formulation comprising;

   (i) a polylactide polymer or poly(lactide-co-glycolide) co-polymer having an average molecular weight of from 5,000 Daltons to 50,000 Daltons;
   (ii) a molar ratio of lactide to glycolide of between 100:0 to 50:50; and
   (iii) from about 10 to 55% by weight of anastrozole based upon the total weight of the formulation

   wherein the prolonged release formulation continuously releases anastrozole over a period of at least 10 days when placed in an aqueous physiological-type environment.

2. A prolonged release formulation according to Claim 1 wherein the prolonged release formulation is a monolithic implant.

3. A prolonged release formulation according to Claim 1 wherein the prolonged release formulation is a microparticle formulation.

4. A prolonged release monolithic implant formulation comprising:

   (i) a polylactide polymer or paly(lactide-co-glycolide) co-polymer having an average molecular weight of from 5,000 Daltons to 50,000 Daltons;
   (ii) a molar ratio of lactide to glycolide of between 100:0 to 50:50;
   (iii) from about 10 to 65% by weight of anastrozole based upon the total weight of the formulation;

   wherein the prolonged release formulation is a monolithic implant and wherein the minimum average molecular weight of the polymer can be calculated from the equation below:

$$MW = 9.937 - 13.0(f_A+0.547) + 5.684(f_A+0.547)^3 - 9.89(f_G-0.177) + 78.95(f_G-0.177)^3 + 83.25(f_A*f_G)$$

   wherein
   $f_A$ is the traction of anastrozole wherein 1.0 is 100%, i.e. 5% would be expressed as 0.05; and
   $f_G$ is the fraction of glycolide wherein 1.0 is 100%, i.e. 50% would be expressed as 0.50.

5. A prolonged release microparticle formulation wherein:

   (i) a polylactide polymer or poly(lactide-co-glycolide) co-polymer having an average molecular weight of from 5,000 Daltons to 50,000 Daltons;
   (ii) a molar ratio of lactide to glycolide of between 100:0 to 50:50; and
   (iii) from about 10 to 55% by weight of anastrozole based upon the total weight of the formulation

   wherein the prolonged release formulation is a microparticle formulation and wherein the minimum average molecular weight of the polymer can be calculated from the equation below:

$$MW = -71.88 + 25.0(0.5 + \tanh(14.3(f_A-0.33))) + 84.5(f_G+1.08) - 12.8(f_G+1.08)^2$$

   wherein
   $f_A$ is the fraction of anastrozole wherein 1.0 is 100%, i.e. 5% would be expressed as 0.05; and
   $f_G$ is the fraction of glycolide wherein 1.0 is 100%, i.e. 50% would be expressed as 0.50.

6. A prolonged release formulation according to any one of the preceding claims wherein the molar ratio of lactide to glycolide is between 100:0 to 95:5.

7. A prolonged release formulation according to any one of the preceding claims wherein the polylactide polymer or poly(lactide-co-glycolide) co-polymer has a weight average molecular weight of from 8,000 Daltons to 45,000 Daltons.

8. A prolonged release monolithic implant formulation according to Claim 2 or Claim 4 wherein the polylactide polymer or poly(lactide-co-glycolide) co-polymer has a weight average molecular weight of from 15,000 Daltons to 30,000 Daltons and the molar ratio of lactide to glycolide is between 100:0 to 95:5.

9. A prolonged release monolithic implant formulation according to Claim 8 when dependent on Claim 4wherein the formulation comprises from about 10 to 60% by weight based upon the total weight of the formulation of anastrozole.

10. A prolonged release microparticle formulation according to Claim 3 or Claim 5 wherein the polylactide polymer or poly(lactide-co-glycolide) co-polymer has a weight average molecular weight of from 10,000 Daltons to 35,000 Daltons and the molar ratio of lactide to glycolide is between 100:0 to 95:5.

11. A prolonged release microparticle formulation according to Claim 10 wherein the formulation comprises from about 10 to 30% by weight based upon the total weight of the formulation of anastrozole.

12. The use of a prolonged release formulation according to any one of Claims 1 to 11 in the manufacture of a medicament for the treatment of a condition, preferably breast cancer, treatable by anastrozole in a warm blooded animal.

13. A process for the preparation of a monolithic implant formulation according to any one or Claims 2 or 4 comprising the steps:

(i) preparing a solution or dispersion of anastrozole in a solution comprising the polylactide or poly(lactide-co-glycolide) polymer and a suitable organic solvent;
(ii) removing substantially all of the organic solvent from the solution or dispersion formed in step (i); and
(iii) forming the product of step (ii) into a monolithic implant of the required dimensions.

14. A process for the preparation of a monolithic implant formulation according to Claim 2 comprising the steps:

(i) preparing a mixture of anastrozole and the polylactide or poly(lactide-co-glycolide) polymer;
(ii) forming the mixture into a monolithic implant of the required dimensions.

15. A process for the preparation of a microparticle formulation according to Claim 3 comprising the steps:

(i) preparing a solution or dispersion of anastrozole in a solution comprising the polylactide or poly(lactide-co-glycolide) polymer and a suitable organic solvent;
(ii) dispersing the solution prepared in (i) into an aqueous processing medium to form an oil-in-water emulsion;
(iii) removing substantially all of the organic solvent from the emulsion formed in step (ii) to form microparticles; and
(iv) washing and drying the microparticles.

**Patentansprüche**

1. Formulierung mit verlängerter Freisetzung, umfassend:

(i) ein Polylactidpolymer oder Poly(lactid-co-glycolid)copolymer mit einem mittleren Molekulargewicht von 5.000 Dalton bis 50.000 Dalton;
(ii) ein Molverhältnis von Lactid zu Glycolid zwischen 100:0 und 50:50; und
(iii) etwa 10 bis 55 Gew.-% Anastrozol, bezogen auf das Gesamtgewicht der Formulierung,

wobei die Formulierung mit verlängerter Freisetzung über einen Zeitraum von wenigstens 10 Tagen kontinuierlich Anastrozol freisetzt, wenn sie in eine wässrige Umgebung vom physiologischen Typ gegeben wird.

2. Formulierung mit verlängerter Freisetzung gemäß Anspruch 1, wobei die Formulierung mit verlängerter Freisetzung ein monolithisches Implantat ist.

3. Formulierung mit verlängerter Freisetzung gemäß Anspruch 1, wobei die Formulierung mit verlängerter Freisetzung eine Mikropartikelformulierung ist.

4. Monolithische Implantatformulierung mit verlängerter Freisetzung, umfassend:

(i) ein Polylactidpolymer oder Poly(lactid-co-glycolid)copolymer mit einem mittleren Molekulargewicht von 5.000 Dalton bis 50.000 Dalton;
(ii) ein Molverhältnis von Lactid zu Glycolid zwischen 100:0 und 50:50; und
(iii) etwa 10 bis 65 Gew.-% Anastrozol, bezogen auf das Gesamtgewicht der Formulierung,

wobei die Formulierung mit verlängerter Freisetzung ein monolithisches Implantat ist, und wobei das minimale mittlere Molekulargewicht des Polymers durch folgende Gleichung berechnet werden kann:

$$MW = 9,937 - 13,0(f_A+0,547) + 5,684(f_A+0,547)^3 - 9,89(f_G-0,177) + 78,95(f_G-0,177)^3 + 83,25(f_A*f_G)$$

wobei
$f_A$ der Anteil von Anastrozol ist, wobei 1,0 100 % entspricht, d.h. 5 % würden als 0,05 ausgedrückt; und
$f_G$ der Anteil von Glycolid ist, wobei 1,0 100 % entspricht, d.h. 50 % würden als 0,5 ausgedrückt.

5. Mikropartikelformulierung mit verlängerter Freisetzung, umfassend:

(i) ein Polylactidpolymer oder Poly(lactid-co-glycolid)copolymer mit einem mittleren Molekulargewicht von 5.000 Dalton bis 50.000 Dalton;
(ii) ein Molverhältnis von Lactid zu Glycolid zwischen 100:0 und 50:50; und
(iii) etwa 10 bis 55 Gew.-% Anastrozol, bezogen auf das Gesamtgewicht der Formulierung,

wobei die Formulierung mit verlängerter Freisetzung eine Mikropartikelformulierung ist, und wobei das minimale mittlere Molekulargewicht des Polymers durch folgende Gleichung berechnet werden kann:

$$MW = -71,88 + 25,0(0,5+\tanh(14,3(f_A-0,33))) + 84,5(f_G+1,08) - 12,8(f_G+1,08)^2$$

wobei
$f_A$ der Anteil von Anastrozol ist, wobei 1,0 100 % entspricht, d.h. 5 % würden als 0,05 ausgedrückt; und
$f_G$ der Anteil von Glycolid ist, wobei 1,0 100 % entspricht, d.h. 50 % würden als 0,5 ausgedrückt.

6. Formulierung mit verlängerter Freisetzung gemäß einem der vorstehenden Ansprüche, wobei das Molverhältnis von Lactid zu Glycolid zwischen 100:0 und 95:5 beträgt.

7. Formulierung mit verlängerter Freisetzung gemäß einem der vorstehenden Ansprüche, wobei das Polylactidpolymer oder Poly(lactid-co-glycolid)copolymer ein gewichtsgemitteltes Molekulargewicht von 8.000 Dalton bis 45.000 Dalton aufweist.

8. Monolithische Implantatformulierung mit verlängerter Freisetzung gemäß Anspruch 2 oder Anspruch 4, wobei das Polylactidpolymer oder Poly(lactid-co-glycolid)copolymer ein gewichtsgemitteltes Molekulargewicht von 15.000 Dalton bis 30.000 Dalton aufweist und das Molverhältnis von Lactid zu Glycolid zwischen 100:0 und 95:5 beträgt.

9. Monolithische Implantatformulierung mit verlängerter Freisetzung gemäß Anspruch 8, abhängig von Anspruch 4, wobei die Formulierung etwa 10 bis 60 Gew.-% Anastrozol, bezogen auf das Gesamtgewicht der Formulierung, umfasst.

10. Mikropartikelformulierung mit verlängerter Freisetzung gemäß Anspruch 3 oder Anspruch 5, wobei das Polylactidpolymer oder Poly(lactid-co-glycolid)copolymer ein gewichtsgemitteltes Molekulargewicht von 10.000 Dalton bis 35.000 Dalton aufweist und das Molverhältnis von Lactid zu Glycolid zwischen 100:0 und 95:5 beträgt.

11. Mikropartikelformulierung mit verlängerter Freisetzung gemäß Anspruch 10, wobei die Formulierung etwa 10 bis 30 Gew.-% Anastrozol, bezogen auf das Gesamtgewicht der Formulierung, umfasst.

12. Verwendung einer Formulierung mit verlängerter Freisetzung gemäß einem der Ansprüche 1 bis 11 bei der Herstellung eines Medikaments zur Behandlung eines Zustands, vorzugsweise Brustkrebs, der durch Anastrozol behandelbar ist, in einem warmblütigen Tier.

13. Verfahren zum Herstellen einer monolithischen Implantatformulierung gemäß einem der Ansprüche 2 oder 4, umfassend die Schritte:

(i) Herstellen einer Lösung oder Dispersion von Anastrozol in einer Lösung, umfassend das Polylactid- oder Poly(lactid-co-glycolid)polymer und ein geeignetes organisches Lösungsmittel;
(ii) Entfernen von im Wesentlichen dem gesamten organischen Lösungsmittel aus der in Schritt (i) gebildeten Lösung oder Dispersion; und
(iii) Ausformen des Produkts von Schritt (ii) zu einem monolithischen Implantat mit den benötigten Abmessungen.

14. Verfahren zum Herstellen einer monolithischen Implantatformulierung gemäß Anspruch 2, umfassend die Schritte:

(i) Herstellen eines Gemischs von Anastrozol und dem Polylactid- oder Poly(lactid-co-glycolid)polymer;
(ii) Ausformen des Gemischs zu einem monolithischen Implantat mit den benötigten Abmessungen.

15. Verfahren zum Herstellen einer Mikropartikelformulierung gemäß Anspruch 3, umfassend die Schritte:

(i) Herstellen einer Lösung oder Dispersion von Anastrozol in einer Lösung, umfassend das Polylactid- oder Poly(lactid-co-glycolid)polymer und ein geeignetes organisches Lösungsmittel;
(ii) Dispergieren der in (i) hergestellten Lösung in einem wässrigen Verarbeitungsmedium, um eine Öl-in-Wasser-Emulsion zu bilden;
(iii) Entfernen von im Wesentlichen dem gesamten organischen Lösungsmittel aus der in Schritt (ii) gebildeten Emulsion, um Mikropartikel zu bilden; und
(iv) Waschen und Trocknen der Mikropartikel.

## Revendications

1. Formule à libération prolongée comprenant :

(i) un polymère de polylactide ou un copolymère de poly(lactide-co-glycolide) de masse moléculaire moyenne comprise entre 5000 daltons et 50 000 daltons ;
(ii) un rapport molaire lactide sur glycolide compris entre 100:0 et 50:50 ; et
(iii) entre environ 10 et 55 % en masse d'anastrozole, par rapport à la masse totale de la formule

la formule à libération prolongée libérant en continu de l'anastrozole sur une durée d'au moins 10 jours lorsqu'elle est placée dans un environnement aqueux de type physiologique.

2. Formule à libération prolongée conforme à la Revendication 1, où la formule à libération prolongée est un implant monolithique.

3. Formule à libération prolongée conforme à la Revendication 1, où la formule à libération prolongée est une formule micro-particulaire.

4. Formule à libération prolongée de type implant monolithique comprenant :

(i) un polymère de polylactide ou un copolymère de poly(lactide-co-glycolide) de masse moléculaire moyenne comprise entre 5000 daltons et 50 000 daltons ;

(ii) un rapport molaire lactide sur glycolide compris entre 100:0 et 50:50 ;
(iii) entre environ 10 et 65 % en masse d'anastrozole, par rapport à la masse totale de la formule ;

la formule à libération prolongée étant un implant monolithique et la masse moléculaire moyenne minimale du polymère pouvant être calculée à partir de l'équation ci-après :

$$MW = 9.937 - 13.0(f_A+0.547) + 5.684(f_A+0.547)^3 - 9.89(f_G-0.177) + 78.95(f_G-0.177)^3 + 83.25(f_A*f_G)$$

où
$f_A$ est la fraction d'anastrozole, 1.0 correspondant à 100 %, c'est-à-dire que 5 % s'écrirait 0.05 ; et
$f_G$ est la fraction de glycolide, 1.0 correspondant à 100 %, c'est-à-dire que 50 % s'écrirait 0.50.

**5.** Formule à libération prolongée de type micro-particulaire comprenant :

(i) un polymère de polylactide ou un copolymère de poly(lactide-co-glycolide) de masse moléculaire moyenne comprise entre 5000 daltons et 50 000 daltons ;
(ii) un rapport molaire lactide sur glycolide compris entre 100:0 et 50:50 ; et
(iii) entre environ 10 et 55 % en masse d'anastrozole, par rapport à la masse totale de la formule

la formule à libération prolongée étant une formule micro-particulaire et la masse moléculaire moyenne minimale du polymère pouvant être calculée à partir de l'équation ci-après :

$$MW = -71.88 + 25.0(0.5 + \tanh(14.3(f_A-0.33))) + 84.5(f_G+1.08) - 12.8(f_G+1.08)^2$$

où
$f_A$ est la fraction d'anastrozole, 1.0 correspondant à 100 %, c'est-à-dire que 5 % s'écrirait 0.05 ; et
$f_G$ est la fraction de glycolide, 1.0 correspondant à 100 %, c'est-à-dire que 50 % s'écrirait 0.50.

**6.** Formule à libération prolongée selon l'une quelconque des revendications précédentes, où le rapport molaire lactide sur glycolide est compris entre 100:0 et 95:5.

**7.** Formule à libération prolongée selon l'une quelconque des revendications précédentes, où le polymère de polylactide ou le copolymère de poly(lactide-co-glycolide) présente une masse moléculaire moyenne en poids comprise entre 8000 daltons et 45 000 daltons.

**8.** Formule à libération prolongée de type implant monolithique conforme à la Revendication 2 ou à la Revendication 4, où le polymère de polylactide ou le copolymère de poly(lactide-co-glycolide) présente une masse moléculaire moyenne en poids comprise entre 15 000 daltons et 30 000 daltons, et le rapport molaire lactide sur glycolide est compris entre 100:0 et 95:5.

**9.** Formule à libération prolongée de type implant monolithique conforme à la Revendication 8 lorsqu'elle dépend de la Revendication 4, où la formule comprend entre environ 10 et 60 % en masse, par rapport à la masse totale de la formule, d'anastrozole.

**10.** Formule à libération prolongée de type micro-particulaire conforme à la Revendication 3 ou à la Revendication 5, où le polymère de polylactide ou le copolymère de poly(lactide-co-glycolide) présente une masse moléculaire moyenne en poids comprise entre 10 000 daltons et 35 000 daltons, et le rapport molaire lactide sur glycolide est compris entre 100:0 et 95:5.

**11.** Formule à libération prolongée de type micro-particulaire conforme à la Revendication 10, où la formule comprend entre environ 10 et 30 % en masse, par rapport à la masse totale de la formule, d'anastrozole.

**12.** Emploi d'une formule à libération prolongée conforme à l'une quelconque des Revendications 1 à 11 dans la fabrication d'un médicament destiné au traitement d'un état pathologique, préférentiellement du cancer du sein, pouvant être traité par l'anastrozole chez un animal à sang chaud.

**13.** Procédé d'élaboration d'une formule de type implant monolithique conforme à l'une quelconque des Revendications 2 où 4, ledit procédé comprenant les étapes suivantes :

(i) préparation d'une solution ou d'une dispersion d'anastrozole dans une solution comprenant le polymère de polylactide ou de poly(lactide-co-glycolide) et un solvant organique adapté ;
(ii) élimination de substantiellement tout le solvant organique de la solution ou de la dispersion obtenue dans l'étape (i) ; et
(iii) mise en forme du produit de l'étape (ii) en un implant monolithique des dimensions requises.

**14.** Procédé d'élaboration d'une formule de type implant monolithique conforme à la Revendication 2, ledit procédé comprenant les étapes suivantes :

(i) préparation d'un mélange d'anastrozole et du polymère de polylactide ou de poly(lactide-co-glycolide) ;
(ii) mise en forme du mélange en un implant monolithique des dimensions requises.

**15.** Procédé d'élaboration d'une formule de type micro-particulaire conforme à la Revendication 3, ledit procédé comprenant les étapes suivantes :

(i) préparation d'une solution ou d'une dispersion d'anastrozole dans une solution comprenant le polymère de polylactide ou de poly(lactide-co-glycolide) et un solvant organique adapté ;
(ii) dispersion de la solution préparée en (i) dans un milieu de traitement aqueux pour former une émulsion huile dans l'eau ;
(iii) élimination de substantiellement tout le solvant organique de l'émulsion formée dans l'étape (ii) pour obtenir des microparticules ; et
(iv) rinçage et séchage des microparticules.

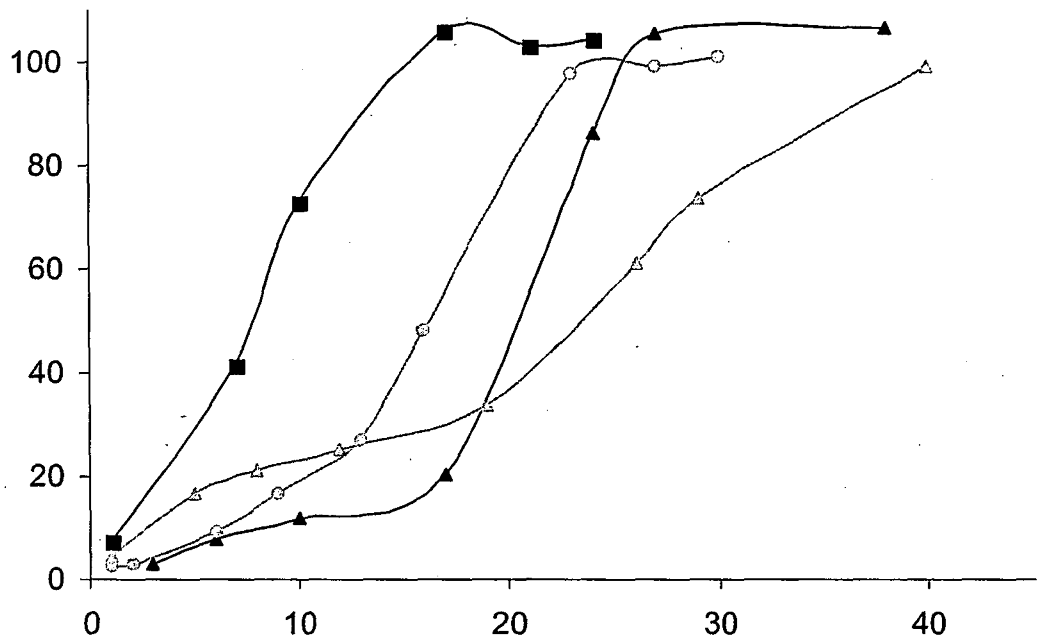


**Figure 1**

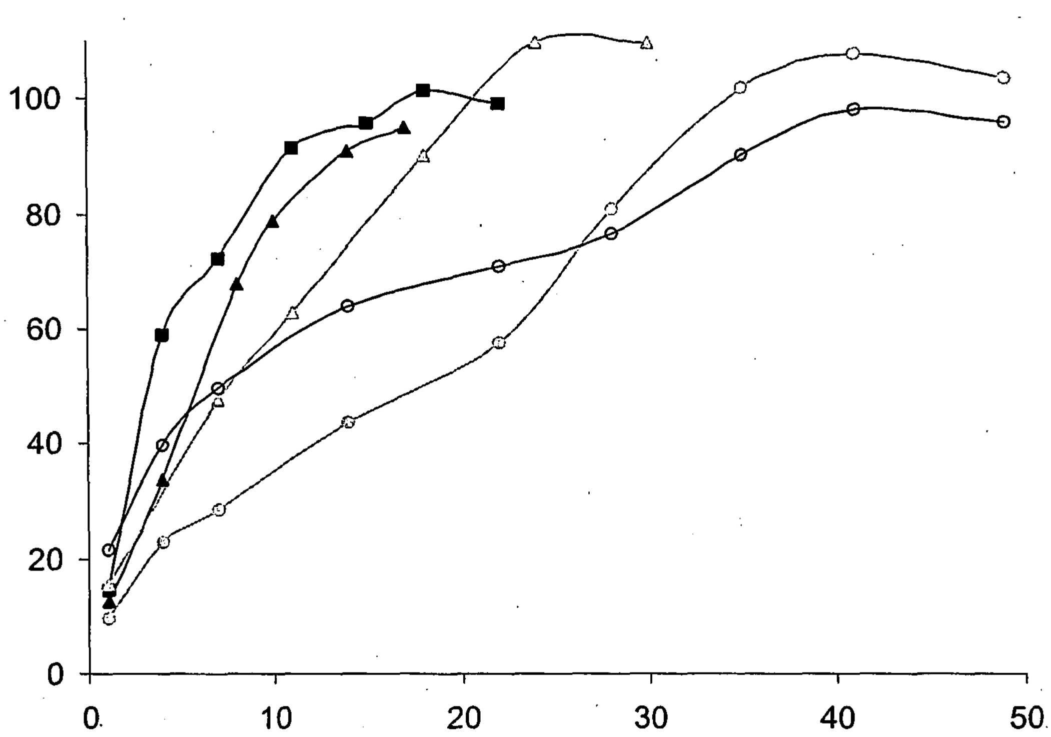


Figure 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US RE366717 E
- EP 058481 A
- US 4384975 A
- US 3891570 A
- US 4389330 A


### Non-patent literature cited in the description


- **Buzdar.** *5 The Breast,* 1995, vol. 4 (3), 256-257
- **Jonat et al.** *European Journal of Cancer,* 1995, vol. 32A (3), 404-412
- **Plourde et al.** *Journal of Steroid Biochemistry,* 1995, vol. 53, 175-179
- Biodegradables and delivery systems for contraception. MTP Press Ltd, 1980
- Controlled Release of Bioactive Agents from Lactide/Glycolide Polymers. **D. H. Lewis.** Biodegradable Polymers as Drug Delivery Systems. Marcel Dekker, Inc, 1990
- Biodegradable Microspheres: Advances in Production Technology. **J. Benoit ; H. Marchais.** Microencapsulation, Methods and Industrial Applications. Marcel Dekker, Inc, 1996
- Biodegradable Polymers: Polyesters. **S. Li ; M. Vert.** Encyclopedia or Controlled Drug Delivery. Wiley-Interscience, 1999, vol. 1
- Polylactic and Polyglycolic Acids as Drug Delivery Carriers. **L. Brannon-Peppas ; M. Vert.** Handbook of Pharmaceutical Controlled Release Technology. Marcel Dekker, Inc, 2000